# EUROPÄISCHE PATENTSCHRIFT

(11) **EP 3 501 583 B1**
(45) Veröffentlichungstag und Bekanntmachung des Hinweises auf die Patenterteilung: **26.05.2021**
(21) Anmeldenummer: 18000988.8
(22) Anmeldetag: 20.12.2018
(51) Int. Cl.: A61G 12/00, G06F 3/14, A61M 16/00

(54) **ATEMTHERAPIESYSTEM**
BREATHING THERAPY SYSTEM
SYSTÈME DE THÉRAPIE RESPIRATOIRE

(30) Priorität: 22.12.2017 DE 102017011940
(43) Veröffentlichungstag der Anmeldung: 26.06.2019
(73) Patentinhaber: Löwenstein Medical Technology S.A., 2557 Luxembourg (LU)
(72) Erfinder: TIEMANN, Björn, 22926 Ahrensburg (DE); GÖBEL, Christof, 22457 Hamburg (DE); WONSYLD, Anne, 20259 Hamburg (DE); REß, Thomas, 25355 Barmstedt (DE); ADAMETZ, Benjamin, 22609 Hamburg (DE); SCHATTNER, Jan, 22041 Hamburg (DE)
(74) Vertreter: Marx, Thomas

(56) Entgegenhaltungen:
- WO-A2-2007/054829
- WO-A2-2008/008659
- WO-A2-2017/144963
- DE-A1- 19 914 749

## Beschreibung

Die vorliegende Erfindung betrifft ein Atemtherapiesystem mit wenigstens zwei separaten und unabhängig voneinander betreibbaren Atemtherapiegeräten zum Betreiben eines solchen Atemtherapiesystems.

Für eine erfolgreiche Beatmung oder Hustenunterstützung ist es in der Regel besonders wichtig, dass die verwendeten Atemtherapiegeräte an die individuellen Bedürfnisse des Patienten angepasst sind. Zudem ist eine möglichst genaue Beobachtung bzw. Überwachung des Therapieverlaufs von großer Bedeutung. Dazu weisen die eingesetzten Geräte oft Bedienelemente und einen integrierten Monitor auf, auf dem der Verlauf bestimmter Beatmungsparameter angezeigt wird. Nachteilig an den bekannten Geräten ist jedoch, dass die Anpassungen an die Bedürfnisse des jeweiligen Patienten häufig sehr aufwendig und zeitintensiv sind. Für Patienten, welche sowohl eine Beatmung als auch eine Hustenunterstützung benötigen, ist der Aufwand noch größer. Zudem ist die Darstellung auf den integrierten Monitoren oft sehr beschränkt und unübersichtlich, da die Geräte möglichst kompakt ausfallen sollen.

Im Stand der Technik sind daher Beatmungsgeräte bekannt geworden, welche an ein Netzwerk angebunden sind. So können mit einem zentralen Computer Einstellungen erfolgen und Daten dargestellt werden. Allerdings bleibt die Bedienung und Auswertung direkt an den Geräten weiterhin eingeschränkt bzw. unübersichtlich.

Ein weiteres Problem ist, dass nicht immer ein entsprechendes Gerät zur Verfügung steht, welches alle für eine optimale Behandlung des Patienten benötigten Funktionen bietet. Dazu müssen entweder sehr viele unterschiedliche Geräte oder sehr umfangreich ausgestattete Geräte bereitstehen, was einen hohen Kostenaufwand mit sich bringt.

Es ist daher die Aufgabe der vorliegenden Erfindung, den Einsatz von Atemtherapiegeräten zu verbessern. Die Erfindung soll dabei insbesondere eine verbesserte Behandlung ermöglichen und zugleich den Aufwand für das Betreiben von Atemtherapiegeräten reduzieren.

Diese Aufgabe wird gelöst durch ein Atemtherapiesystem mit den Merkmalen des Anspruchs 1. Bevorzugte Weiterbildungen sind Gegenstand der Unteransprüche. Weitere Vorteile und Merkmale der vorliegenden Erfindung ergeben sich aus der allgemeinen Beschreibung und der Beschreibung der Ausführungsbeispiele. Das erfindungsgemäße Atemtherapiesystem umfasst wenigstens zwei separate und unabhängig voneinander betreibbare Atemtherapiegeräte. Die Atemtherapiegeräte weisen jeweils wenigstens eine Steuereinrichtung und jeweils wenigstens eine mittels der Steuereinrichtung ansteuerbare Gerätekomponente zur Bereitstellung wenigstens einer Gerätefunktion auf. Dabei ist wenigstens eine Koppeleinrichtung zur Herstellung einer Wirkverbindung zwischen den wenigstens zwei Atemtherapiegeräten vorgesehen. Die Koppeleinrichtung ist dazu geeignet und ausgebildet, bei hergestellter Wirkverbindung die Steuereinrichtungen der wenigstens zwei Atemtherapiegeräte anzusteuern. Mittels der Koppeleinrichtung ist wenigstens eine Gerätekomponente des wenigstens einen Atemtherapiegerätes durch wenigstens eine Gerätekomponente des wenigstens einen anderen Atemtherapiegerätes wenigstens teilweise funktionell erweiterbar und/oder wenigstens teilweise funktionell ersetzbar.

Das erfindungsgemäße Atemtherapiesystem bietet viele Vorteile. Ein erheblicher Vorteil ist, dass die Gerätefunktion eines Atemtherapiegerätes durch eine Gerätefunktion des anderen Atemtherapiegerätes ergänzt bzw. ersetzt werden kann. So müssen die bereitstehenden Geräte nicht immer über alle benötigten Funktionen verfügen, da sie sich entsprechend ergänzen können. So kann zum Beispiel ein Gerät mit weniger Funktionen mit einem Gerät mit einer gerade benötigten Funktion gekoppelt werden, um eine optimale Versorgung des Patienten zu erreichen. Da nicht zwei oder mehrere Geräte mit dem vollen Funktionsumfang vorgehalten werden müssen, können erhebliche Kosten eingespart werden. Zugleich kann die Versorgung des Patienten funktionell erweitert werden. Ein besonderer Vorteil der Erfindung ist zudem, dass die Atemtherapiegeräte auch alleine bzw. unabhängig voneinander eingesetzt werden können. Zudem kann durch die funktionelle Ergänzung bzw. Erweiterung die Bedienung und Auswertung erheblich vereinfacht werden.

Besonders bevorzugt umfasst das Atemtherapiesystem wenigstens zwei verschiedene Arten von Atemtherapiegeräten. Insbesondere sind die wenigstens zwei Atemtherapiegeräte unterschiedlich ausgebildet und umfassen vorzugsweise unterschiedliche Gerätekomponenten und/oder unterschiedliche Gerätefunktionen. Die wenigstens zwei Atemtherapiegeräte können auch gleichartig bzw. identisch sein.

Vorzugsweise umfasst das Atemtherapiesystem wenigstens ein als Beatmungsgerät ausgebildetes Atemtherapiegerät und wenigstens ein als Hustengerät ausgebildetes Atemtherapiegerät. Durch die Ausstattung mit solchen Geräten bietet das Atemtherapiesystem besonders viele Vorteile. So kann eine optimale Versorgung des Patienten bei der Beatmung und bei einer Hustenunterstützung bzw. Hustenmanövern erfolgen, ohne dass beide Geräte einen vollen Funktionsumfang aufweisen müssen. Das Atemtherapiesystem kann auch wenigstens eine andere Art eines Atemtherapiegerätes umfassen.

Insbesondere wird das eine Atemtherapiegerät durch das Beatmungsgerät und das andere Atemtherapiegerät durch das Hustengerät bereitgestellt. Möglich und bevorzugt ist auch, dass das eine Atemtherapiegerät durch das Hustengerät und das andere Atemtherapiegerät durch das Beatmungsgerät bereitgestellt wird. Die wenigstens zwei Atemtherapiegeräte können aber auch als zwei Hustengeräte oder als zwei Beatmungsgeräte ausgebildet sein.

Das Hustengerät dient insbesondere zur gezielten Unterstützung einer Sekretabfuhr aus den Atemwegen eines Patienten. Das Hustengerät umfasst insbesondere wenigstens eine Husteneinrichtung zum Erzeugen wenigstens einer Atemluftströmung für eine Insufflation in den Patienten und/oder wenigstens eine Atemluftströmung für eine Exsufflation aus dem Patienten. Das Beatmungsgerät umfasst insbesondere wenigstens eine Beatmungseinrichtung zum Erzeugen wenigstens einer Atemluftströmung zur Beatmung eines Patienten. Die Husteneinrichtung und/oder die Beatmungseinrichtung umfasst insbesondere wenigstens eine Gebläseeinrichtung und/oder eine Vorrichtung zur Druckvorgabe und/oder eine Vorrichtung zur Volumenvorgabe.

Erfindungsgemäß wird wenigstens eine Anzeigeeinrichtung des wenigstens einen Atemtherapiegerätes durch wenigstens eine Anzeigeeinrichtung des wenigstens einen anderen Atemtherapiegerätes wenigstens teilweise funktionell erweitert und/oder ersetzt . Beispielsweise erfolgen Einstellungen von Parametern an einem Gerät während die Anzeige über ein Display des anderen Geräts ausgegeben wird. So wird eine Datenvisualisierung erweitert bzw. ersetzt. Dadurch ist eine erheblich verbesserte Darstellung von Daten bzw. Informationen direkt an den Atemtherapiegeräten möglich. So muss der Betreuer nicht erst zu einem Rechner mit einem entsprechend großen Monitor wechseln, um eine übersichtliche Darstellung der Daten zu bekommen.

Die Anzeigeeinrichtung dient insbesondere zur Datenvisualisierung. Die Anzeigeeinrichtung umfasst insbesondere wenigstens ein Display und/oder wenigstens einen Monitor und/oder wenigstens einen Bildschirm oder dergleichen. Die Anzeigeeinrichtung kann auch wenigstens eine Bedieneinrichtung bereitstellen und ist dann vorzugsweise als ein Touchscreen oder dergleichen ausgebildet.

Insbesondere dient die Anzeigeeinrichtung zur Visualisierung von Geräteparametern und/oder Beatmungsparametern. Insbesondere sind auf den gekoppelten Anzeigeeinrichtungen unterschiedliche Datenvisualisierungen darstellbar. Insbesondere dient wenigstens ein Monitor des wenigstens einen Atemtherapiegerätes als zusätzlicher Monitor für das wenigstens eine Atemtherapiegerät. Beispielsweise dient der Monitor des Hustengeräts als zusätzlicher Monitor für das Beatmungsgerät. Es ist möglich, dass die Anzeigeeinrichtungen der wirkverbundenen und/oder separaten Atemtherapiegeräte jeweils unter Berücksichtigung einer räumlichen Lage des Atemtherapiegerätes ausrichtbar sind bzw. ausgerichtet werden. So kann der Benutzer das Gerät für sich optimal aufstellen, ohne dass er dadurch Einbußen bei der Betrachtung der Anzeigeeinrichtung in Kauf nehmen muss. Die Ausrichtung der Anzeigeeinrichtungen erfolgt insbesondere automatisch. Möglich ist auch, dass die Ausrichtung nach einer Eingabe einer Information über die räumliche Lage erfolgt. Vorzugsweise ist wenigstens eine Bedieneinrichtung des wenigstens einen Atemtherapiegerätes durch wenigstens eine Bedieneinrichtung des wenigstens einen anderen Atemtherapiegerätes wenigstens teilweise funktionell erweiterbar und/oder ersetzbar. Das hat den Vorteil, dass beispielsweise eine Bedienung bzw. Steuerung oder auch eine Dateneingabe des einen Atemtherapiegerätes durch eine Bedienung bzw. Steuerung oder Dateneingabe des anderen Atemtherapiegerätes ergänzt bzw. ersetzt werden kann. Die Bedieneinrichtung umfasst insbesondere wenigstens ein Eingabegerät bzw. ein Human Interface Device. Die Bedieneinrichtung umfasst beispielsweise wenigstens einen Schalter und/oder wenigstens eine Tastatur und/oder Maus und/oder wenigstens einen Touchscreen und/oder wenigstens ein Touchpad oder dergleichen. Die Bedieneinrichtung kann wenigstens teilweise in eine Anzeigeeinrichtung integriert sein.

Die ersetzte Bedieneinrichtung ist insbesondere deaktivierbar. Insbesondere erfolgt eine automatische Deaktivierung bei Herstellung der Wirkverbindung. Möglich ist auch, dass die Deaktivierung der Bedieneinrichtung nach einer Benutzereingabe erfolgt. Es kann auch eine zeitgleiche Bedienung beider Bedieneinrichtungen vorgesehen sein.

Insbesondere ist die Bedieneinrichtung eines Beatmungsgeräts durch eine Bedieneinrichtung eines Hustengeräts erweiterbar und/oder ersetzbar. Möglich ist auch, dass eine Bedieneinrichtung eines Hustengeräts durch eine Bedieneinrichtung eines Beatmungsgeräts erweiterbar und/oder ersetzbar ist.

Insbesondere ist von der Bedieneinrichtung des wenigstens einen Atemtherapiegerätes aus eine Therapieeinrichtung des wenigstens einen anderen Atemtherapiegerätes ansteuerbar. Insbesondere umfasst die Therapieeinrichtung wenigstens eine Beatmungseinrichtung und/oder wenigstens eine Husteneinrichtung. So wird die Einstellung der Therapieeinrichtung erheblich komfortabler. Insbesondere umfasst auch das eine Atemtherapiegerät wenigstens eine Therapieeinrichtung, welche insbesondere wenigstens eine Husteneinrichtung und/oder wenigstens eine Beatmungseinrichtung umfasst.

Insbesondere ist die Therapieeinrichtung des jeweiligen Atemtherapiegerätes auch von dessen Bedieneinrichtung aus ansteuerbar. Möglich ist aber auch, dass die Bedienbarkeit der Therapieeinrichtung des jeweiligen Atemtherapiegerätes von dessen Bedieneinrichtung aus deaktiviert ist bzw. deaktivierbar ist.

Insbesondere ist von der Bedieneinrichtung des einen Atemtherapiegerätes aus die Therapieeinrichtung des anderen Atemtherapiegerätes aktivierbar und/oder deaktivierbar und/oder einstellbar. Beispielsweise ist ein Start und/oder ein Stopp einer Therapie und beispielsweise einer Beatmungsfunktion und/oder Hustenfunktion durchführbar. Beispielsweise sind auch Einstellungen von Geräteparametern und/oder Beatmungsparametern vornehmbar. Möglich ist auch, dass eine Automatikfunktion und/oder eine manuelle Funktion auswählbar ist. Insbesondere sind der Zeitpunkt eines Starts und/oder Stopps und/oder einer Pause und/oder andere entsprechende Parameter einstellbar. Insbesondere ist wenigstens eine Therapieeinrichtung des wenigstens einen Atemtherapiegerätes durch wenigstens eine Therapieeinrichtung des wenigstens einen anderen Atemtherapiegerätes wenigstens teilweise funktionell erweiterbar und/oder ersetzbar. Dabei umfasst eine der beiden Therapieeinrichtungen insbesondere wenigstens eine Beatmungseinrichtung. Die andere der beiden Therapieeinrichtungen umfasst insbesondere wenigstens eine Husteneinrichtung. Möglich ist auch, dass beide Therapieeinrichtungen eine Beatmungseinrichtung und/oder eine Husteneinrichtung umfassen. So können sich Atemtherapiegeräten funktionell ergänzen, um dem Patienten einen optimalen Therapieumfang zu bieten.

Vorzugsweise ist mittels der Koppeleinrichtung die Therapieeinrichtung des wenigstens einen Atemtherapiegerätes mit der Therapieeinrichtung des wenigstens einen anderen Atemtherapiegerätes wenigstens unter Berücksichtigung wenigstens eines Geräteparameters und/oder Beatmungsparameters synchronisierbar. Dadurch kann der Aufwand einer patientenspezifischen Anpassung der Geräte erheblich vereinfacht werden. So muss zum Beispiel nur ein Gerät auf den Patienten eingestellt werden, während das andere Atemtherapiegerät über die Koppeleinrichtung synchronisiert und angepasst wird. Beispielsweise ist es besonders hilfreich, eine Husteneinrichtung mit den Einstellungen einer Beatmungseinrichtung zu synchronisieren. Besonders vorteilhaft ist auch, dass die Synchronisation direkt an einem oder beiden Atemtherapiegeräten erfolgen kann. So kann auf einen Zugriff über einen entfernt stehenden Rechner verzichtet werden. Alternativ kann die Synchronisierung auch über einen entfernt stehenden Rechner, der benötigte Daten vorhält und abgleicht, erfolgen. Insbesondere ist eine Husteneinrichtung mit einer Beatmungseinrichtung synchronisierbar oder umgekehrt. Insbesondere ist wenigstens ein Geräteparameter und/oder Beatmungsparameter von dem einen zum anderen Atemtherapiegerät oder umgekehrt übertragbar. Insbesondere ist dabei ein Zugriff von der einen Therapieeinrichtung auf die andere Therapieeinrichtung möglich, um den Geräteparameter und/oder Beatmungsparameter abzurufen und/oder einzustellen. Insbesondere erfolgt die Synchronisation bei hergestellter Wirkverbindung. Möglich ist eine wenigstens teilweise automatische und/oder wenigstens teilweise manuelle Synchronisation. Beispielsweise kann auswählbar sein, welcher Geräteparameter und/oder Beatmungsparameter synchronisiert werden soll. Möglich ist auch, dass der synchronisierte Geräteparameter und/oder Beatmungsparameter wenigstens teilweise modifiziert bzw. angepasst werden kann.

Die Therapieeinrichtung des wenigstens einen Atemtherapiegerätes ist vorzugsweise dazu geeignet und ausgebildet, mittels der Koppeleinrichtung wenigstens eine in dem wenigstens einen anderen Atemtherapiegerät angeordnete Verneblereinrichtung wenigstens teilweise anzusteuern und insbesondere zu deaktivieren und/oder zu aktivieren und/oder einzustellen. So kann die Verneblereinrichtung für beide Geräte eingesetzt werden, ohne in beiden Geräten tatsächlich eingebaut zu sein. Die Verneblereinrichtung ist insbesondere auch vom Atemtherapiegerät aus ansteuerbar. Beispielsweise ist die Verneblereinrichtung in einer Beatmungseinrichtung eines Beatmungsgerätes angeordnet. Dabei ist die Verneblereinrichtung vorzugsweise durch eine Husteneinrichtung eines Hustengerätes ansteuerbar und zur Therapie einsetzbar. Es kann dazu auch eine andere Komponente als die Verneblereinrichtung vorgesehen sein. Besonders bevorzugt ist die Therapieeinrichtung des wenigstens einen Atemtherapiegerätes dazu geeignet und ausgebildet, mittels der Koppeleinrichtung ein Ausatemsystem des wenigstens einen anderen Atemtherapiegerätes anzusteuern und insbesondere pneumatisch anzusteuern. Das Ausatemsystem ist insbesondere an dem anderen Atemtherapiegerät angeschlossen. Insbesondere ist von einem Beatmungsgerät aus ein Ausatemsystem eines Hustengerätes ansteuerbar oder umgekehrt. Durch eine solche funktionelle Kopplung kann die Atemtherapie erheblich verbessert werden.

Vorzugsweise ist die Therapieeinrichtung des wenigstens einen Atemtherapiegerätes mit wenigstens einem Exspirationsteil bzw.

Expirationsblock des anderen Atemtherapiegerätes zur Herstellung einer Strömungsverbindung verbindbar, um bei hergestellter Wirkverbindung als eine Unterdruckeinheit für die Ausatmung in der Exspiration zu dienen. Der Einsatz als Unterdruckeinheit für die Ausatmung in der Exspiration kann zeitabhängig und/oder eingabeabhängig erfolgen. Der Exspirationsteil ist insbesondere Teil der Therapieeinrichtung des anderen Atemtherapiegerätes. Dabei ist das eine Atemtherapiegerät insbesondere ein Hustengerät. Das andere Atemtherapiegerät ist insbesondere als ein Beatmungsgerät ausgebildet. Möglich ist auch eine umgekehrte Ausgestaltung. Insbesondere ist eine Husteneinrichtung mit dem Exspirationsteil des Beatmungsgeräts verbindbar.

In einer besonders vorteilhaften Ausgestaltung ist die Koppeleinrichtung dazu geeignet und ausgebildet, die Gerätekomponente des wenigstens einen Atemtherapiegerätes und/oder die Gerätekomponente des wenigstens einen anderen Atemtherapiegerätes wenigstens teilweise zu deaktivieren und/oder zu aktivieren, wenn die Atemtherapiegeräte wirkverbunden sind. So kann der Betrieb einer Gerätekomponente eingespart werden, wenn eine Wirkverbindung vorliegt. Dadurch kann zum Beispiel eine Akkulaufzeit erheblich verlängert werden. Beispielsweise kann eine Gebläseeinrichtung oder Anzeigeeinrichtung oder Bedieneinrichtung teilweise deaktiviert werden, wenn das wirkverbundene andere Gerät eine entsprechende Komponente bereitstellt.

Insbesondere ist die Gerätekomponente durch die Koppeleinrichtung deaktivierbar, welche durch die andere Gerätekomponente wenigstens teilweise ersetzt und/oder erweitert ist. Zum Deaktivieren kann beispielsweise ein Standby und/oder ein Ruhemodus und/oder vollständiges Abschalten vorgesehen sein. Es ist möglich, dass das Deaktivieren und/oder Aktivieren automatisch und/oder manuell und beispielsweise mittels einer Benutzereingabe durchführbar ist.

Vorzugsweise findet eine Aktivierung statt, sobald die Nutzung der deaktivierten Gerätekomponente erkannt und/oder angefordert wird. Zum Beispiel ist eine Wake Up Funktion vorgesehen, beispielsweise von einer Bedieneinrichtung aus. Insbesondere wird die Gerätekomponente aktiviert, wenn eine Anforderung und/oder Bedienung von dem Atemtherapiegerät mit der deaktivierten Gerätekomponente aus erfolgt.

In einer besonders bevorzugten Ausgestaltung umfasst die Koppeleinrichtung wenigstens eine Aufnahmeeinrichtung zur physischen Aufnahme der wenigstens zwei Atemtherapiegeräte. Vorzugsweise weist die Aufnahmeeinrichtung für die Atemtherapiegeräte jeweils wenigstens eine Aufnahmeeinheit auf. Möglich und bevorzugt ist auch, dass die Aufnahmeeinrichtung für die Atemtherapiegeräte wenigstens eine gemeinsame Aufnahmeeinheit aufweist. So kann mit den wirkverbundenen Geräten besonders komfortabel und ergonomisch gearbeitet werden. Es ist möglich, dass mit Aufnahme der Atemtherapiegeräte in die Aufnahmeeinrichtung eine automatische Herstellung der Wirkverbindung erfolgt und/oder wenigstens eine Benutzereingabe für die Herstellung der Wirkverbindung abgefragt wird. Beispielsweise können die Atemtherapiegeräte und die Aufnahmeeinrichtung mit einer Funktion in der Art eines Plug and Play ausgestattet sein. Es ist möglich, dass die Aufnahmeeinrichtung dazu geeignet und ausgebildet ist, die Art des aufgenommenen Atemtherapiegerätes automatisch zu erkennen. Die Kopplung kann auch eine Wirkverbindung ohne eine physische Kopplung der Atemtherapiegeräte umfassen.

Die Aufnahmeeinheiten und/oder die gemeinsame Aufnahmeeinheit kann als eine Wandhalterung und/oder Betthalterung und/oder Rollstuhlhalterung und/oder Tischhalterung und/oder als ein Fahrgestell oder dergleichen ausgebildet sein.

Es ist möglich, dass die in der Aufnahmeeinrichtung aufgenommen Atemtherapiegeräte übereinander und/oder nebeneinander und/oder Rücken an Rücken anordenbar sind. Solche Anordnungen bieten einen besonders guten Zugang bzw. eine besonders praxistaugliche Nutzung der Geräte. Es können auch andere geeignete Anordnungen, beispielsweise hintereinander oder dergleichen, vorgesehen sein. Insbesondere ist eine der wenigstens zwei Anzeigeeinrichtungen deaktivierbar bzw. deaktiviert, wenn die Atemtherapiegeräte Rücken an Rücken angeordnet sind. Insbesondere erfolgt eine automatische Deaktivierung und/oder es wird wenigstens eine Benutzereingabe abgefragt, wenn der Kopplungsmodus Rücken an Rücken erkannt wird.

Insbesondere sind die Anzeigeeinrichtungen in verschiedene Richtungen und insbesondere zu gegenüberliegenden Seiten ausgerichtet, wenn die Atemtherapiegeräte Rücken an Rücken angeordnet sind. Insbesondere sind die Anzeigeeinrichtungen im Wesentlichen in eine gemeinsame Richtung ausgerichtet, wenn die Atemtherapiegeräte übereinander und/oder nebeneinander und/oder hintereinander angeordnet sind.

Das Verfahren dient zum Betreiben eines Atemtherapiesystems mit wenigstens zwei separaten und unabhängig voneinander betreibbaren Atemtherapiegeräten. Die Atemtherapiegeräte weisen jeweils wenigstens eine Steuereinrichtung und jeweils wenigstens eine durch die Steuereinrichtung angesteuerte Gerätekomponente auf. Durch die Gerätekomponente wird wenigstens eine Gerätefunktion bereitgestellt. Dabei wird mittels wenigstens einer Koppeleinrichtung wenigstens eine Wirkverbindung zwischen den wenigstens zwei Atemtherapiegeräten hergestellt. Bei hergestellter Wirkverbindung werden die Steuereinrichtungen der wenigstens zwei Atemtherapiegeräte wenigstens zeitweise angesteuert. Wenigstens eine Gerätekomponente des wenigstens einen Atemtherapiegerätes wird durch wenigstens eine Gerätekomponente des wenigstens einen anderen Atemtherapiegerätes wenigstens teilweise funktionell erweitert und/oder wenigstens teilweise funktionell ersetzt.

Auch das Verfahren bietet viele Vorteile.

Besonders vorteilhaft ist, dass die gekoppelten Atemtherapiegeräte funktionell erweitert bzw. ergänzt werden. So wird eine erheblich verbesserte Funktionalität und Behandlungsqualität als bei separat eingesetzten Geräten erreicht.

Insbesondere dient das Verfahren zum Betreiben des zuvor beschriebenen Atemtherapiesystems. Insbesondere ist das erfindungsgemäße Atemtherapiesystem dazu geeignet und ausgebildet, nach dem Verfahren betrieben zu werden.

Vorzugsweise werden Daten des einen Atemtherapiegerätes auf wenigstens einer Anzeigeeinrichtung des anderen Atemtherapiegerätes visualisiert, sodass wenigstens eine Anzeigeeinrichtung des einen Atemtherapiegerätes ergänzt und/oder ersetzt wird. Insbesondere werden Daten eines Beatmungsgerätes auf einer Anzeigeeinrichtung eines Hustengerätes visualisiert.

Insbesondere wird eine hergestellte bzw. aktive Wirkverbindung mittels wenigstens einer Grafik und/oder wenigstens eines Symbols in der Anzeigeeinrichtung des einen und/oder des anderen Atemtherapiegerätes angezeigt. Dadurch erhält der Benutzer einen Hinweis, dass zwei Anzeigeeinrichtungen aktiv sind oder welche Anzeigeeinrichtung ihm zur Verfügung steht. Dabei betreffen die Daten vorzugsweise wenigstens einen Geräteparameter und/oder wenigstens einen Beatmungsparameter einer Beatmung eines Patienten. Der Geräteparameter und/oder der Beatmungsparameter werden vorzugsweise grafisch dargestellt. Insbesondere wird eine grafische Auftragung des Beatmungsparameters und/oder des Geräteparameters als eine Funktion der Zeit und/oder als Funktion eines anderen Bezugsparameters dargestellt. Beispielsweise wird eine Kurvenansicht dargestellt. Insbesondere werden zwei oder drei oder mehr Geräteparameter und/oder Beatmungsparameter grafisch dargestellt. Möglich ist auch eine numerische Darstellung und/oder eine Darstellung in Textform.

Es ist möglich, dass Daten des einen Atemtherapiegerätes zugleich auch auf dessen Anzeigeeinrichtungen visualisiert werden. Möglich ist auch, dass die Anzeigeeinrichtung des einen Atemtherapiegerätes wenigstens teilweise deaktiviert ist oder zur Anzeige anderer Informationen bereitgestellt wird. Insbesondere dient wenigstens eine Anzeigeeinrichtung zur grafischen Darstellung einer Verlaufsfunktion wenigstens eines Geräteparameters und/oder Beatmungsparameters. Insbesondere dient dann die andere Anzeigeeinrichtung zur numerischen und/oder schriftlichen Darstellung wenigstens eines Geräteparameters und/oder Beatmungsparameters.

In einer besonders vorteilhaften Ausgestaltung wird auf dem einen Atemtherapiegerät wenigstens ein Menü zur Steuerung einer Beatmungseinrichtung dargestellt. Insbesondere erfolgt zugleich auf dem anderen Atemtherapiegerät eine grafische Darstellung des Beatmungsparameters. Besonders bevorzugt erfolgt auf dem anderen Atemtherapiegerät eine grafische Auftragung des Beatmungsparameters als eine Funktion der Zeit und/oder als Funktion eines anderen Beatmungsparameters. Dabei ist das andere Atemtherapiegerät vorzugsweise als ein Hustengerät ausgebildet oder umgekehrt. Eine solche Ausgestaltung hat den Vorteil, dass die Anzeigeeinrichtungen bzw. ein Monitor des Hustengerätes zur Visualisierung von Daten des Beatmungsgerätes eingesetzt werden kann. So kann die Beatmungseinrichtung über ein besonders übersichtliches Menü eingestellt werden und zugleich kann eine zuverlässige Überwachung der Beatmungsparameter erfolgen. Wird das Hustengerät dann beispielsweise für ein Hustenmanöver eingesetzt, kann automatisch oder manuell eine Umschaltung erfolgen, sodass auf der Anzeigeeinrichtung des Hustengeräts ein Menü zu dessen Steuerung oder dergleichen angezeigt wird.

In einer Ausgestaltung kann auf einem als Hustengerät ausgebildeten Atemtherapiegerät ein Menü zur Steuerung einer Husteneinrichtung dargestellt werden. Dabei kann auf dem anderen Atemtherapiegerät, welches insbesondere als Beatmungseinrichtung ausgebildet ist, zugleich eine grafische Darstellung eines für das Hustenmanöver spezifischen Beatmungsparameters und/oder Geräteparameters erfolgen. Eine solche Ausgestaltung bietet eine vorteilhafte Ergänzung der Anzeige Einrichtung des Hustengerätes mit der Anzeige Einrichtung des Beatmungsgerätes.

Möglich ist auch, dass auf beiden Anzeigeeinrichtungen ein Menü zur Steuerung einer Beatmungseinrichtung und/oder einer Husteneinrichtung dargestellt wird. Möglich ist auch, dass auf beiden Anzeigeeinrichtungen eine grafische Darstellung des Beatmungsparameters und/oder Geräteparameters erfolgt. Möglich ist, dass mittels einer Benutzereingabe ausgewählt wird, auf welchem der beiden Atemtherapiegeräte das Menü zur Steuerung bzw. die grafische Darstellung des Beatmungsparameters erfolgen soll.

Insbesondere wird eine Bedienung des einen Atemtherapiegerätes auf einer Bedieneinrichtung des anderen Atemtherapiegerätes durchgeführt, sodass eine Bedieneinrichtung des einen Atemtherapiegerätes ergänzt und/oder ersetzt wird. Insbesondere erfolgt eine Bedienung eines Beatmungsgerätes auf einer Bedieneinrichtung eines Hustengerätes. Möglich ist auch, dass eine Bedienung eines Hustengerätes auf einer Bedieneinrichtung eines Beatmungsgerätes durchgeführt wird. Das bietet eine vorteilhafte Anpassung des Umfangs der Bedieneinrichtung, sodass Einstellungen und Eingaben sehr sicher und komfortabel vorgenommen werden können.

Es ist möglich, dass das eine Atemtherapiegerät wenigstens eine Fernbedienung umfasst. Dabei wird das andere Atemtherapiegerät vorzugsweise mittels der Fernbedienung bedient. Beispielsweise ist ein Beatmungsgerät mit einer Fernbedienung ausgestattet. Ist das Beatmungsgerät mit einem Hustengerät gekoppelt, kann vorzugsweise auch ein Hustenmanöver mittels der Fernbedienung ausgelöst werden. Möglich ist auch eine umgekehrte Ausgestaltung.

Vorzugsweise wird von dem einen Atemtherapiegerät aus wenigstens eine Gerätefunktion des anderen Atemtherapiegerätes gestartet und/oder gestoppt und/oder eingestellt. Das kann manuell über eine Bedieneinrichtung oder automatisch über einen entsprechenden Steuerbefehl erfolgen. Beispielsweise wird von einem Beatmungsgerät aus ein Hustenmanöver eines Hustengerätes gestartet und/oder gestoppt und/oder eingestellt. Möglich ist auch, dass von einem Hustengerät aus eine Beatmungsfunktion eines Beatmungsgeräts gestartet und/oder gestoppt und/oder eingestellt wird.

Dabei kann insbesondere während des Hustenmanövers mittels der Anzeigeeinrichtung des Beatmungsgerätes und/oder des Hustengerätes ein Verlauf von Druck und/oder Flow und/oder Volumen und/oder Sauerstoffsättigung visualisiert werden. Möglich ist auch, dass während einer Beatmung mittels der Anzeigeeinrichtung des Hustengerätes und/oder des Beatmungsgerätes ein Verlauf von Druck und/oder Flow und/oder Volumen und/oder Sauerstoffsättigung visualisiert wird.

Es ist möglich und bevorzugt, dass von dem einen Atemtherapiegerät aus wenigstens ein Geräteparameter und/oder Beatmungsparameter für eine Gerätefunktion an das andere Atemtherapiegerät übertragen wird. Vorzugsweise wird dabei die Gerätefunktion des anderen Atemtherapiegerätes in Abhängigkeit des übertragenen Geräteparameters bzw. Beatmungsparameters angepasst, sodass eine Synchronisation bzw. ein Parametersetting der Atemtherapiegeräte ermöglicht wird. So kann ein gekoppeltes Atemtherapiegerät sehr zügig und zugleich sehr zuverlässig an die individuellen Bedürfnisse eines Patienten angepasst werden.

Das Parametersetting ist dann besonders vorteilhaft, wenn ein Patient beispielsweise sowohl mit einem Hustengerät als auch mit einem Beatmungsgerät versorgt werden muss. Dann wäre im Stand der Technik die Einstellung von zwei Geräten notwendig, was einen entsprechenden Zeit- und Arbeitsaufwand mit sich bringt. Durch die hier vorgestellte Erfindung ist es möglich, das zweite Gerät schnell und sicher durch das Parametersetting einzustellen. Ist der Patient beispielsweise mit einem Beatmungsgerät versorgt und soll noch ein Hustengerät ergänzt werden, ist dadurch für das Hustengerät keine aufwendige Einstellung mehr notwendig.

Zusätzlich oder alternativ kann die Übertragung des Geräteparameters und/oder des Beatmungsparameters in die andere Richtung erfolgen. Möglich ist auch eine beidseitige Übertragung bzw. eine gegenseitige Synchronisation. Die Gerätefunktion betrifft insbesondere eine Therapiefunktion und zum Beispiel ein Hustenmanöver und/oder eine Beatmung. Möglich sind auch andere Gerätefunktionen, wie zum Beispiel Anzeigeeinstellungen, Bedieneinstellungen und/oder Konfigurationseinstellungen. Vorzugsweise wird ein Hustenmanöver bzw. eine Hustentherapie in Abhängigkeit einer Beatmung angepasst bzw. synchronisiert. Möglich ist auch, dass eine Beatmung in Abhängigkeit der Hustentherapie angepasst bzw. synchronisiert wird. Insbesondere nimmt das Beatmungsgerät wenigstens ein Parametersetting an einem Hustengerät vor. Möglich ist auch, dass das Hustengerät wenigstens ein Parametersetting an einem Beatmungsgerät vornimmt.

In allen Ausgestaltungen ist es bevorzugt, dass eine Husteneinrichtung eines als Hustengerät ausgebildeten Atemtherapiegerätes von einem als Beatmungsgerät ausgebildeten Atemtherapiegerät angesteuert und/oder bedient wird. Insbesondere wird während der Beatmung von der Husteneinrichtung wenigstens eine Atemunterstützung bereitgestellt. Die Atemunterstützung ist insbesondere als ein Hustenmanöver ausgebildet oder umfasst wenigstens ein solches.

So kann die Beatmung eines Patienten erheblich verbessert werden. Möglich ist auch, dass während eines Hustenmanövers und/oder zwischen wenigstens zwei Hustenmanövern eine Atemunterstützung mittels einer Beatmungseinrichtung des Beatmungsgerätes bereitgestellt wird.

In einer vorteilhaften Ausgestaltung wird von dem Beatmungsgerät aus wenigstens ein Zeitpunkt einer Insufflation und/oder einer Exsufflation und/oder einer Pause eingestellt. Vorzugsweise wird die Husteneinrichtung von dem Beatmungsgerät aus in wenigstens einen Automatikmodus geschaltet, bei dem ein Hustenmanöver wenigstens teilweise automatisch ausgelöst wird. Die Husteneinrichtung kann von dem Beatmungsgerät aus auch wenigstens teilweise manuell bedient werden, sodass Zeitpunkt und/oder Dauer und/oder andere Parameter des Hustenmanövers durch wenigstens eine Benutzereingabe einstellbar sind. Während der Ansteuerung und/oder Bedienung des Hustengerätes von dem Beatmungsgerät aus kann eine Bedienung am Hustengerät selbst deaktiviert bzw. gesperrt sein. Es kann auch eine parallele Bedienung des Hustengerätes von beiden Geräten aus vorgesehen sein.

In einer vorteilhaften Ausgestaltung führt das Beatmungsgerät während einer Beatmung wenigstens teilweise automatisch mittels der Husteneinrichtung wenigstens ein Hustenmanöver durch. Möglich ist auch, dass das Beatmungsgerät insbesondere während einer Beatmung wenigstens einen Hinweis an einen Benutzer ausgibt, welcher auf ein durchzuführendes bzw. anstehendes Hustenmanöver hinweist. Nach dem Hinweis kann automatisch ein Hustenmanöver erfolgen. Möglich ist auch, dass nach dem Hinweis eine Benutzereingabe abgewartet wird.

Zeitlich vor dem Hustenmanöver kann wenigstens eine in dem Beatmungsgerät angeordnete Verneblereinrichtung aktiviert werden, um das Abhusten durch Vernebelung insbesondere von Medikamenten vorzubereiten. So kann ein erheblich angenehmeres und wirksameres Abhusten erfolgen. Zudem wird eine zweite Verneblereinrichtung eingespart.

In einer bevorzugten Ausgestaltung wird durch das Beatmungsgerät und das Hustengerät jeweils wenigstens ein Geräteparameter und/oder Beatmungsparameter über die Zeit registriert. Insbesondere werden die registrierten Geräteparameter bzw. Beatmungsparameter zwischen dem Hustengerät und dem Beatmungsgerät in wenigstens eine Richtung ausgetauscht. Dabei wird vorzugsweise wenigstens eine gemeinsame Visualisierung der Geräteparameter bzw. Beatmungsparameter vor und/oder nach und/oder während eines Hustenmanövers angezeigt, sodass der Effekt des Hustenmanövers überwacht werden kann. Zusätzlich oder alternativ zur gemeinsamen Visualisierung kann auch eine andere geeignete Auswertung der registrierten Daten vorgesehen sein.

In einer bevorzugten Weiterbildung wird in den miteinander wirkverbundenen bzw. gekoppelten Atemtherapiegeräten jeweils wenigstens ein Hinweis auf die Kopplung angezeigt. Insbesondere wird dazu die jeweilige Anzeigeeinrichtung eingesetzt. Vorzugsweise wird an wenigstens einem der wenigstens zwei Atemtherapiegeräte ein Menü zur Anpassung der Kopplung bereitgestellt. Beispielsweise ist eine Eingabe als Bestätigung erforderlich, um die Kopplung aufrechtzuerhalten und/oder um die Kopplung zu deaktivieren. Vorzugsweise wird der Hinweis bei Entkopplung wieder ausgeblendet. Vorzugsweise werden bei einer Entkopplung insbesondere auch deaktivierte Gerätekomponenten wieder aktiviert. Insbesondere werden bei Entkopplung die Anzeigeeinrichtung und/oder die Bedieneinrichtung wieder aktiv, wenn diese zuvor deaktiviert wurden.

Vorzugsweise wird in dem Menü wenigstens ein Kopplungsmodus aus einer Gruppe von Kopplungsmodi ausgewählt. Die Kopplungsmodi beschreiben insbesondere räumliche und/oder funktionale Anordnungen der gekoppelten Atemtherapiegeräte. Vorzugsweise umfasst die Gruppe wenigstens eine Anordnung der gekoppelten Atemtherapiegeräte übereinander und nebeneinander und Rücken an Rücken. Die Gruppe kann auch eine Anordnung hintereinander und andere Anordnungen umfassen.

Es ist möglich, dass für die zu koppelnden Atemtherapiegeräte wenigstens eine Aufnahmeeinrichtung vorgesehen ist. Die Aufnahmeeinrichtung kann eine mittelbare und/oder auch eine unmittelbare Verbindung der beiden Atemtherapiegeräte umfassen. Dabei kann eine Kopplung automatisch dann erfolgen, wenn die Atemtherapiegeräte in die Aufnahmeeinrichtung eingesetzt werden.

Die Koppeleinrichtung ist insbesondere dazu geeignet und ausgebildet, wenigstens die Steuereinrichtungen der wenigstens zwei Atemtherapiegeräte derart anzusteuern, dass die Gerätekomponente des einen Atemtherapiegerätes durch die Gerätekomponente des anderen Atemtherapiegerätes wenigstens teilweise funktionell erweiterbar und/oder ersetzbar ist. Insbesondere ist die Koppeleinrichtung dazu geeignet und ausgebildet, wenigstens eine Gerätefunktion des einen Atemtherapiegerätes mittels wenigstens einer Gerätekomponente des anderen Atemtherapiegerätes wenigstens teilweise bereitzustellen.

Insbesondere sind die Atemtherapiegeräte mittels der Koppeleinrichtung miteinander wirkverbindbar. Die Koppeleinrichtung umfasst insbesondere wenigstens eine Schnittstelle zur Wirkverbindung der Atemtherapiegeräte.

Beispielsweise ist eine elektronische Datenübertragungsschnittstelle vorgesehen. Die Datenübertragung kann drahtlos und/oder drahtgebunden erfolgen. Die Koppeleinrichtung ist insbesondere mit den Gerätekomponenten wirkverbunden. Insbesondere kann die Koppeleinrichtung die Steuereinrichtungen derart ansteuern, dass mittels der Gerätekomponenten die entsprechenden Gerätefunktionen bereitgestellt werden können.

Die Atemtherapiegeräte sind insbesondere mit jeweils einer Gehäuseeinrichtung ausgestattet. Unter separaten Atemtherapiegeräten werden insbesondere Atemtherapiegeräte mit jeweils einer Gehäuseeinrichtung verstanden. Insbesondere können die beiden Atemtherapiegeräte separat und unabhängig voneinander an jeweils einem Patienten eingesetzt werden. Insbesondere sind die Atemtherapiegeräte unabhängig und separat voneinander transportierbar.

Die Gerätekomponente umfasst insbesondere wenigstens eine Anzeigeeinrichtung und/oder Bedieneinrichtung und/oder Therapieeinrichtung und vorzugsweise wenigstens eine Beatmungseinrichtung und/oder Husteneinrichtung oder ist als eine solche ausgebildet.

Im Rahmen der vorliegenden Erfindung wird unter einem Beatmungsparameter vorzugsweise auch ein Hustenparameter bzw. ein Parameter einer Hustentherapie verstanden. Der Beatmungsparameter ist beispielsweise ein Druck oder ein Flow oder ein Volumen oder dergleichen. Der Beatmungsparameter kann auch solche Größen als Funktion der Zeit oder als Funktion eines anderen Bezugsparameters beschreiben. Der Beatmungsparameter kann auch beschreibend für die Atemtätigkeit des Patienten sein, beispielsweise das Atemvolumen oder dergleichen. Der Beatmungsparameter kann auch direkt oder indirekt am Patienten sensorisch erfasst werden.

Im Rahmen der vorliegenden Erfindung wird unter einem Geräteparameter insbesondere eine Größe verstanden, die an dem Atemtherapiegerät einstellbar ist bzw. eingestellt wird. Beispielsweise beschreibt der Geräteparameter eine Drehzahl eines Gebläses der Beatmungseinrichtung oder der Husteneinrichtung. Der Geräteparameter kann auch eine andere am Gerät einstellbare Größe oder eine Steuergröße für eine Gerätekomponente beschreiben. Der Geräteparameter kann auch eine Konfigurationseinstellung umfassen.

Die Anmelderin behält sich vor, ein Atemtherapiegerät für das zuvor beschriebene Atemtherapiesystem zu beanspruchen. Vorzugsweise ist das Atemtherapiegerät wie zuvor beschrieben ausgebildet. Insbesondere ist das Atemtherapiegerät als ein Hustengerät oder ein Beatmungsgerät ausgebildet. Das Atemtherapiegerät ist insbesondere mit einem Beatmungsgerät oder einem Hustengerät koppelbar.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigt:
- Fig. 1: eine stark schematische Darstellung eines erfindungsgemäßen Atemtherapiesystems;
- Fig. 2: eine stark schematische Darstellung eines Kopplungsmodus eines Atemtherapiesystems; und
- Fig. 3-5: stark schematische Darstellungen weiterer Kopplungsmodi.

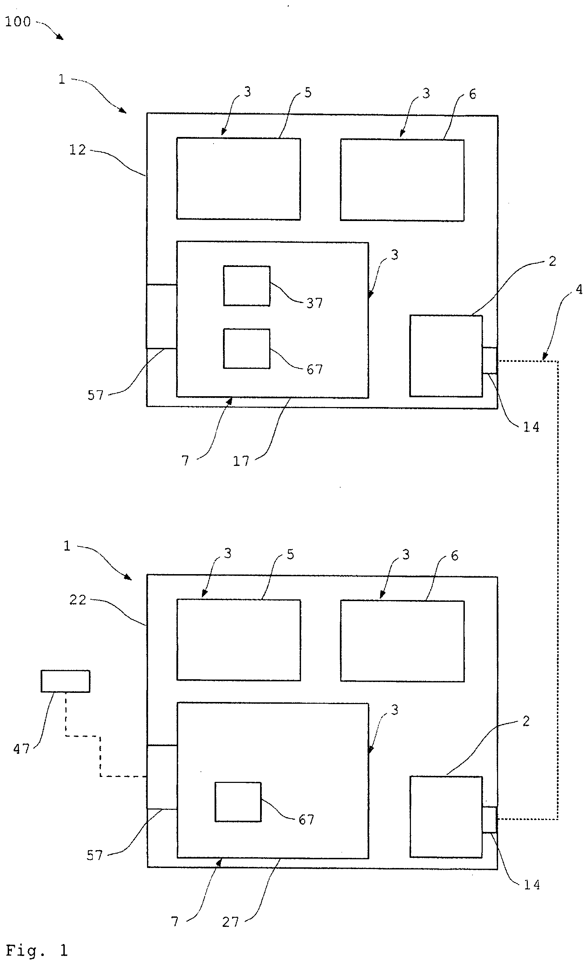

Die Figur 1 zeigt ein erfindungsgemäßes Atemtherapiesystem 100, welches hier zwei Atemtherapiegeräte 1 umfasst. Das Atemtherapiesystem 100 wird nach dem Verfahren betrieben. Die Atemtherapiegeräte 1 werden hier durch ein Beatmungsgerät 12 und ein Hustengerät 22 bereitgestellt. Das Beatmungsgerät 12 umfasst hier eine Therapieeinrichtung 7, welche als Beatmungseinrichtung 17 ausgebildet ist. Die Beatmungseinrichtung 17 ist mit einer Gebläseeinrichtung 67 bzw. einem Lüfter ausgestattet, um eine Atemluftströmung zur Beatmung des Patienten zu erzeugen. Die Beatmungseinrichtung 17 kann zusätzlich zumindest ein Ventil umfassen. Die Beatmungseinrichtung 17 umfasst beispielsweise zumindest eine Gebläseeinrichtung 67 und optional oder ergänzend zumindest ein Ventil mit welchen eine Atemluftströmung für eine Insufflation und eine Atemluftströmung für eine Exsufflation erzeugt wird. Die Beatmungseinrichtung 17 umfasst alternativ beispielsweise zwei Gebläseeinrichtungen 67 und zumindest ein Ventil mit welchen eine Atemluftströmung für eine Insufflation und eine Atemluftströmung für eine Exsufflation erzeugt wird. Die Gebläseeinrichtung und/oder das Ventil sind optional zusätzlich ausgebildet und eingerichtet eine Atemluftströmung für eine Insufflation und/oder eine Atemluftströmung für eine Exsufflation mit einer definierten Pulsation zu überlagern. Die Gebläseeinrichtung und/oder das Ventil sind optional zusätzlich ausgebildet und eingerichtet die Atemluftströmung für eine Exsufflation in Form eines Unterdruckes zu erzeugen.

Über eine Anschlusseinrichtung 57 kann ein hier nicht näher dargestelltes Atemschlauchsystem angeschlossen werden. Zudem umfasst die Beatmungseinrichtung 17 hier beispielsweise eine Verneblereinrichtung 37, um zum Beispiel Medikamente in der Atemluft zu vernebeln.

Das Hustengerät 22 dient zur gezielten Unterstützung einer Sekretabfuhr aus den Atemwegen eines Patienten und umfasst hier eine als Husteneinrichtung 27 ausgebildete Therapieeinrichtung 7. Die Husteneinrichtung 27 umfasst beispielsweise zumindest eine Gebläseeinrichtung 67 und optional ergänzend zumindest ein Ventil mit welchen eine Atemluftströmung für eine Insufflation und eine Atemluftströmung für eine Exsufflation erzeugt wird. Die Husteneinrichtung 27 umfasst alternativ beispielsweise zwei Gebläseeinrichtungen 67 und zumindest ein Ventil mit welchen eine Atemluftströmung für eine Insufflation und eine Atemluftströmung für eine Exsufflation erzeugt wird. Die Gebläseeinrichtung und/oder das Ventil sind optional zusätzlich ausgebildet und eingerichtet eine Atemluftströmung für eine Insufflation und/oder eine Atemluftströmung für eine Exsufflation mit einer definierten Pulsation zu überlagern, um Sekret zu lösen. Die Gebläseeinrichtung und/oder das Ventil sind optional zusätzlich ausgebildet und eingerichtet die Atemluftströmung für eine Exsufflation in Form eines Unterdruckes zu erzeugen, um so den Hustenstoß eines Patienten effektiv zu unterstützen.

Über eine Anschlusseinrichtung 57 kann ein hier nicht näher dargestelltes Schlauchsystem angeschlossen werden, welches für Hustenmanöver bzw. eine Hustentherapie geeignet ist. Das Schlauchsystem umfasst zum Beispiel ein Ausatemsystem 47. Darüber kann beispielsweise kontinuierlich Ausatemluft abführbar sein. Das Abführen von Ausatemluft kann aber auch gezielt an Atemphasen bzw. Hustenphasen angepasst werden.

Um neben einer Therapiefunktion weitere Gerätefunktionen zu ermöglichen, sind die Atemtherapiegeräte 1 hier mit weiteren Gerätekomponenten 3 ausgestattet. So weist jedes Atemtherapiegerät 1 hier eine Anzeigeeinrichtung 5 und eine Bedieneinrichtung 6 zur Vornahme von Eingaben und Einstellungen auf. Die Anzeigeeinrichtung 5 umfasst zum Beispiel einen Monitor bzw. Display. Es kann auch ein Touchscreen vorgesehen sein. Die Atemtherapiegeräte 1 können weitere, hier nicht näher dargestellte Gerätekomponenten 3 aufweisen.

Zur Ansteuerung der Gerätekomponenten 3 umfassen die Atemtherapiegeräte 1 hier jeweils eine oder mehrere Steuereinrichtungen 2. Die Gerätekomponenten 3 können auch teilweise eigene bzw. separate Steuereinrichtungen aufweisen. In der Steuereinrichtung 2 sind vorzugsweise Vorgaben zur Ansteuerung der Gerätekomponenten 3 hinterlegt bzw. gespeichert. Diese Vorgaben können insbesondere durch den Benutzer bzw. einen Betreuer wenigstens teilweise angepasst werden. Die Steuereinrichtung 2 umfasst beispielsweise wenigstens einen Controller und/oder andere Steuerkomponenten. Das Atemtherapiesystem 100 weist eine Koppeleinrichtung 4 auf, um eine Wirkverbindung zwischen den Atemtherapiegeräten 1 herstellen zu können. Für die Koppeleinrichtung 4 kann zum Beispiel in jedem Atemtherapiegerät 1 eine Schnittstelle 14 bzw. Connecting Interface angeordnet sein. Über die Schnittstellen 14 können die Atemtherapiegeräte 1 drahtlos und/oder drahtgebunden kommunizieren. Die Schnittstellen 14 sind vorzugsweise mit den Steuereinrichtungen 2 wirkverbunden. Die Koppeleinrichtung 4 ist dazu geeignet und ausgebildet, bei hergestellter Wirkverbindung die Steuereinrichtungen 2 der Atemtherapiegeräte 1 anzusteuern. Die Koppeleinrichtung 4 kann kabelgebunden oder kabellos ausgebildet sein beispielsweise als USB oder Bluetooth Verbindung. Die Koppeleinrichtung 4 kann alternativ auch als Steckverbindung zwischen den Atemtherapiegeräten ausgebildet sein.

Das Atemtherapiesystem 100 bietet hier die Möglichkeit, über die Koppeleinrichtung 4 eine oder mehrere Gerätekomponenten 3 eines Atemtherapiegerätes 1 durch eine entsprechende Gerätekomponente 3 des anderen Atemtherapiegerätes 1 funktionell zu erweitern oder zu ersetzen.

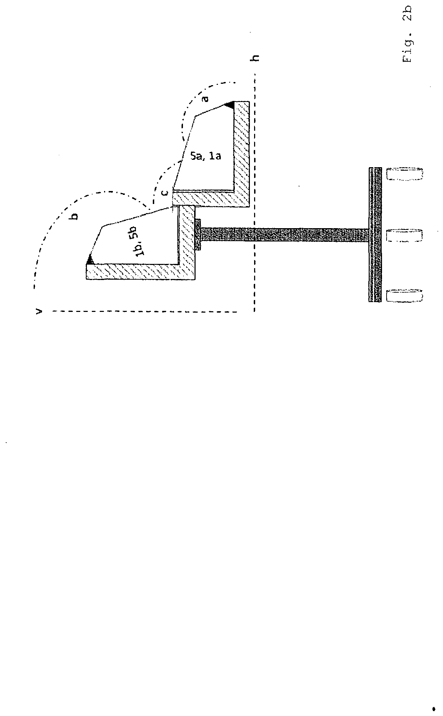

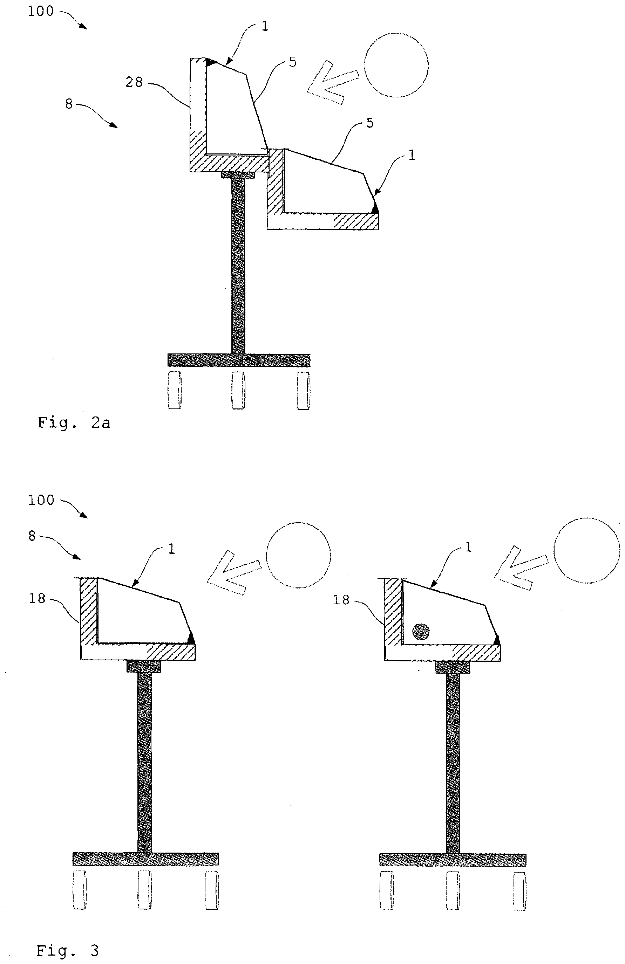

In den Figuren 2 bis 5 ist das Atemtherapiesystem 100 mit gekoppelten Atemtherapiegeräten 1 gezeigt, welche in verschiedenen Kopplungsmodi angeordnet sind. Die Betrachterposition und der Blickwinkel eines Benutzers sind durch einen Kreis bzw. einen Pfeil skizziert.

In der Figur 2a ist eine Aufnahmeeinrichtung 8 zur physischen Aufnahme der Atemtherapiegeräte 1 in einer gemeinsamen Aufnahmeeinheit 28 gezeigt. Die Aufnahmeeinrichtung 8 kann auch zur mittelbaren physischen Verbindung der Atemtherapiegeräte 1 mit der Aufnahmeeinheit 28 ausgebildet sein. Die Atemtherapiegeräte 1 weisen dann identische, mechanische Verbindungselemente auf, die eine mechanische Kopplung mit Kopplungsmitteln der Aufnahmeeinheit 28 ermöglichen. Die mechanischen Verbindungselemente sind beispielsweise weiblich ausgeführt und die Kopplungsmittel sind männlich ausgeführt. Ergänzend können die mechanischen Verbindungselemente und die Kopplungsmittel mechanische Rastmittel aufweisen. Die Aufnahmeeinheit 28 ist hier mit einem Fahrgestell ausgestattet. Ein Atemtherapiegerät 1 ist hier aufrecht positioniert, während das andere Atemtherapiegerät 1 liegend angeordnet ist. Die Aufnahmeeinrichtung 8 kann auch zur unmittelbaren physischen Verbindung der Atemtherapiegeräte 1 ausgebildet sein. Die Atemtherapiegeräte 1 weisen dann zueinander komplementäre, mechanische Verbindungselemente auf, die eine mechanische Kopplung beider Atemtherapiegeräte 1 ermöglichen.

Die Anzeigeeinrichtungen sind beispielsweise mit Lagesensoren ausgestattet, sodass die Anzeigeeinrichtungen die Darstellungsweise an die Positionierung des Atemtherapiegerätes anpassen.

In dieser Anordnung wird ein besonderer Vorteil deutlich. Die Figur 2b zeigt: Die Anzeigeeinrichtung 5a des einen Atemtherapiegerätes 1a ist in einem Winkel (a) zu einer Horizontalen (h) geneigt angeordnet. Dieser Winkel ist liegt im Bereich 4° bis 25°, bevorzugt bei 6° bis 18°, besonders bevorzugt bei 9° bis 15° zur Horizontalen.

Die Anzeigeeinrichtung 5b des anderen Atemtherapiegerätes 1b ist in einem Winkel (b) zu einer Vertikalen (v) geneigt angeordnet. Dieser Winkel ist liegt im Bereich 4° bis 25°, bevorzugt bei 6° bis 18°, besonders bevorzugt bei 9° bis 15° zur Vertikalen. Für einen Betrachter (B) sind daher beide Anzeigeeinrichtungen gut ablesbar.

Die Anzeigeeinrichtungen 5a und 5b weisen einen Winkel (c) zueinander auf, der größer als 90° ist, bevorzugt bei 92° bis 102°, besonders bevorzugt bei 92° bis 132°, ganz besonders bevorzugt bei 92° bis 162°. Für einen Betrachter (B) sind daher beide Anzeigeeinrichtungen gemeinsam gut ablesbar.

Die Atemtherapiegeräte sind bevorzugt so ausgerichtet, dass die Unterkante einer Anzeigeeinrichtung im Wesentlichen auf derselben Höhe ist wie die Oberkante der anderen Anzeigeeinrichtung.

Die Anzeigeeinrichtungen 5 können dabei an die jeweilige Lage ausgerichtet werden. In diesem Kopplungsmodus sind zum Beispiel beide Anzeigeeinrichtungen 5 aktiv, sodass eine erweiterte Darstellung erfolgen kann.

In der Figur 3 ist eine Aufnahmeeinrichtung 8 gezeigt, welche für jedes Atemtherapiegerät 1 jeweils eine Aufnahmeeinheit 18 mit einem Fahrgestell umfasst.

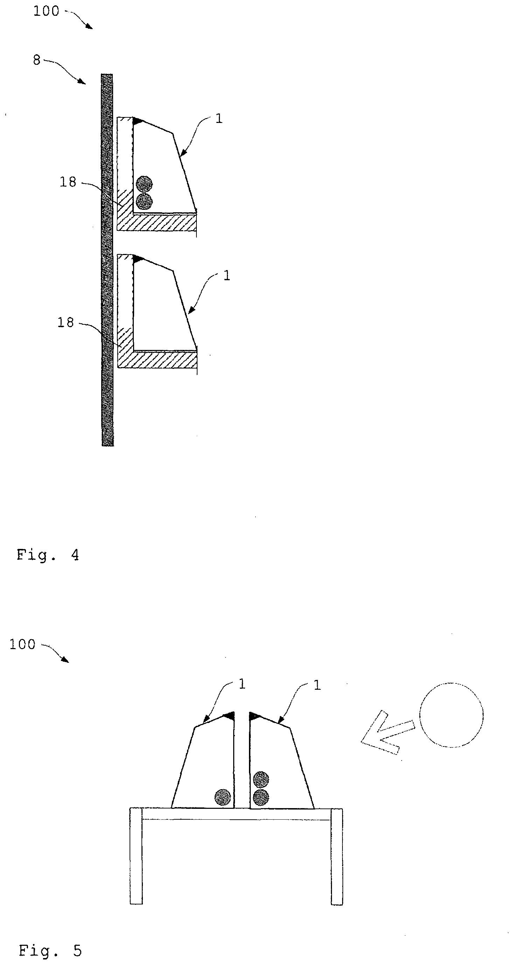

Die Figur 4 zeigt die Aufnahmeeinheiten 18 in einer Wandmontage. Dabei können die Atemtherapiegeräte 1 zum Beispiel übereinander oder nebeneinander angeordnet werden.

In der Figur 5 ist ein Kopplungsmodus ohne eine physische Kopplung der Atemtherapiegeräte 1 gezeigt. Zum Beispiel stehen die Atemtherapiegeräte 1 dabei auf einem Tisch oder dergleichen. Die Atemtherapiegeräte 1 sind hier Rücken an Rücken angeordnet. Dabei ist vorzugsweise eine der Anzeigeeinrichtungen 5 deaktiviert.

In einem Beispiel wird das Beatmungsgerät 12 mit dem Hustengerät 22 zur Steuerung, Datenvisualisierung und Datenanalyse gekoppelt.

In einem Beispiel wird mit dem Hustengerät 22 zur Steuerung, Datenvisualisierung und Datenanalyse gekoppelt.

Daten von dem Hustengerät 22 können so auf das Beatmungsgerät 12 übertragen werden, wobei der Datenspeicher des Beatmungsgerätes 12 für Daten von dem Beatmungsgerät 12 und für Daten von dem Hustengerät 22 genutzt wird. Das Beatmungsgerät liefert dabei eine Zeitinformation, die mit den Daten gespeichert wird. Sofern das Hustengerät ebenfalls Daten mit einer Zeitinformation liefert, wird diese Zeitinformation des Hustengerätes auf die Zeitinformation des Beatmungsgerätes normiert. Die so chronologisch gespeicherten Daten von Beatmungsgerät und Hustengerät können von einer Software ausgelesen und visualisiert werden oder per Datenfernübertragung transferiert werden oder von einem Nutzer ausgelesen und verarbeitet werden. Durch die gemeinsame Auswertung von Beatmungs- und Hustendaten in einer Software ist es möglich, die Auswirkungen der Therapie besser zu beurteilen. Beispielsweise wird ein Patient beatmet und die physiologischen Werte verschlechtern sich (bemerkbar an geringerem Inspirationsvolumen und geringer Sauerstoffsättigung (Sp02)). Dann wird ein Hustenmanöver durchgeführt und evtl. festsitzendes Sekret gelöst. Dass die Hustentherapie erfolgreich war, kann bspw. an dem gemessenen Spitzenfluss in der Ausatmung (PCF) festgestellt werden. Danach wird wieder beatmet und die physiologischen Werte werden wieder gemessen. Dadurch kann beobachtet werden, welchen Einfluss was für ein Hustenmanöver hatte.

So kann das Hustengerät 22 als zusätzlicher Monitor für das Beatmungsgerät 12 dienen. Das Hustengerät 22 kann mit dem Beatmungsgerät 12 verbunden werden und dient als zusätzlicher Monitor, in dem verschiedene Beatmungsparameter angezeigt werden können. Dabei werden beide Geräte zum Beispiel fest und insbesondere lösbar auf einem Fahrgestell oder einer Wandhalterung montiert.

Es können auf unterschiedlichen Anzeigeeinrichtungen unterschiedliche Daten dargestellt werden, um insgesamt mehr Daten darzustellen.
Beispielsweise können Daten von dem Beatmungsgerät 12 wie Drücke, Flow, Volumen, Sauerstoffgehalt im Blut jeweils über der Zeit, aber auch Druck über Flow oder Druck über Volumen von dem Beatmungsgerät aufgezeichnet werden und auf der Anzeigeeinrichtung 5 sowohl des Beatmungsgerät 12 und des Hustengerätes dargestellt werden. Dabei ist bevorzugt vorgesehen, dass bestimmte Daten und Messwerte von dem Beatmungsgerät auf der Anzeigeeinrichtung 5 des Beatmungsgerätes dargestellt werden und andere Daten und Messwerte des Beatmungsgerätes auf der Anzeige des Hustengerätes dargestellt werden.
Auf den beiden Anzeigeeinrichtungen können jeweils spezifische für den Betrachter relevante Daten dargestellt werden. Für den ärztlichen Fachmann können beispielsweise Druck und Flowkurven auf der Anzeigeeinrichtung 5 des Beatmungsgerätes 12 dargestellt werden und für den Patienten auf der der Anzeigeeinrichtung 5 des Hustengerätes vereinfachte Signale oder Symbole (bspw. Pfeile) für die Inspiration und Exspiration.

Die Erfindung bietet den Vorteil, dass die Informationen je nach Betrachter räumlich getrennt und separat aufbereitet werden können. Anders als bei nur einem Monitor (der die Werte nur wiedergibt), können sie hier auch entsprechend verarbeitet werden.
Im Besonderen sind unterschiedliche Ebenen möglich, die die Bedienung ergänzen. Es können beide Anzeigeeinrichtungen bedient werden, um mit den Daten zu interagieren, beispielsweise durch ein tippen und stoppen oder zoomen in den jeweiligen Bereich.

So kann zum Beispiel eine Kurvenansicht der Beatmung ohne Überlagerung des Ventilator-Monitors erfolgen.

Es kann zum Beispiel ein Starten und Stoppen eines automatischen Modus vom Beatmungsgerät 12 aus erfolgen, sodass das Hustengerät 22 nicht bedient werden muss. Zudem kann auch der manuelle Modus von dem Beatmungsgerät 12 aus gestartet werden, sodass dann mithilfe einer Fernbedienung das Hustenmanöver ausgelöst werden kann. Währenddessen wird auf dem Beatmungsgerät 12 der Signalverlauf von Druck und Flow gezeigt. Es kann auch das Hustenmanöver selbst vom Beatmungsgerät 12 aus bedient werden. Das bedeutet das Bestimmen der Zeitpunkte der Insufflation, Exsufflation und Pause durch eine Bedienung direkt am Beatmungsgerät 12.

Zum Beispiel kann ein Parametersetting für das Hustengerät 22 in dem Beatmungsgerät 12 erfolgen. Es erfolgt die Einstellung / Steuerung verschiedener Parameter der Hustengerät 22 mittels des Beatmungsgerätes 12. Es kann bspw. die Übertragung der eingestellten Beatmungsparameter für die Atemunterstützung in der Hustengerät 22 erfolgen.

Weiterhin kann eine zeitbasierte oder individuelle, an den Anwender angepasste Benachrichtigung / Erinnerung über das Beatmungsgerät 12 erfolgen, die auf eine anstehende / erneute Durchführung der therapeutischen Atemunterstützung mittels Hustengerät 22 hinweist, So kann beispielsweise eine sensorische Erfassung von Parametern - wie Fluss, Druck, Sauerstoffsättigung, Frequenz und Volumen - während der Beatmung und Auswertung dieser Parameter individuell für den Patienten erfolgen. Aus einem Vergleich dieser Parameter-Werte über einen bestimmten Zeitraum, von Stunden oder Tagen oder Wochen, kann das Beatmungsgerät 12 eine mögliche Verschlechterung der Patientenatmung identifizieren und daraufhin einen Warnhinweis oder eine Empfehlung generieren das Hustengerät 22 einzusetzen.

Potenziell kann auch ein integrierter Vernebler 37 aktiviert werden, um das Abhusten durch Medikamentenverneblung vorzubereiten.

Die Abschaltung der Hustengerät 22 kann ebenfalls über das Beatmungsgerät 12 erfolgen (Energiesparmodus).

Ein besonderer Vorteil ist, dass nur ein Gerät verwendet bzw. bedient wird. Das bietet eine platzsparende Aufstellung, Vereinfachung der Husten-Anwendung.

Es kann auch eine gemeinsame Datenvisualisierung mit einem Datentransfer von der Hustengerät 22 auf das Beatmungsgerät 12 und damit eine bessere Auswertung der Signalverläufe eines Patienten im Zusammenhang beider Therapien. Zum Beispiel kann ein potenzieller positiver Effekt des Abhustens von Sekret auf die folgenden Beatmungswerte (vor allem Volumen) besser im Zusammenhang betrachtet werden (Vorher / Nachher-Visualisierung).

Bei der Kopplung bzw. wenn die Geräte 1 verbunden werden, erscheint zum Beispiel in beiden Geräten 1 ein Kopplungssymbol.

In beiden Geräten 1 erscheint zum Beispiel eine Benachrichtigung, dass die Kopplung hergestellt wurde. In einem der Geräte 1 und zum Beispiel im Beatmungsgerät 12 bietet eine Benachrichtigung bzw. Benutzereingabe die Möglichkeit, den Kopplungsmodus zu ändern. So ist der Kopplungsmodus zum Beispiel in einem System-Menü einstellbar.

Die Verbindung kann in mehreren Kopplungsmodi umgesetzt werden. Bei Kopplung ist vorzugsweise immer der zuletzt verwendete Kopplungsmodus aktiv.

Zum Beispiel ist bei einer Rücken an Rücken Anordnung der Monitor bzw. Bildschirm eines Geräts 1 und insbesondere des Hustengeräts 22 ausgeschaltet. Die Therapie des Hustengeräts 22 lässt sich dann über das Beatmungsgerät 12 oder die Fernbedienung und zum Beispiel einen Gehäuseschalter starten und beenden. In der Anzeige des Beatmungsgeräts 12 steht die Funktion bereit, das Hustengerät 22 fern zu steuern. Die Funktion bildet zum Beispiel den Homebildschirm des Hustengeräts 22 nach. Nun kann die Hustentherapie gestartet und gestoppt werden. Es kann zum Beispiel das Therapieprogramm des Hustengeräts 22 gewählt oder eingestellt werden. Es werden Therapiemesswerte der Hustengerät angezeigt. In der Ereignisliste des Beatmungsgeräts 12 werden der Start und der Stopp der Hustentherapie vermerkt. Dabei kann gleichzeitig die Ventilation bzw. Beatmung des Beatmungsgeräts 12 aktiv sein. Zum Beispiel sind bei einer Anordnung der Geräte 1 übereinander oder nebeneinander beide Bildschirme aktiv. Beide Geräte 1 sind in ihrer gewohnten weise nutzbar. Auf dem Bildschirm des Beatmungsgeräts 12 steht die Funktion bereit, den Bildschirm zu erweitern. Ist die Funktion deaktiviert, ist in der Anzeige des Hustengeräts 22 dessen Benutzeroberfläche nutzbar. Ist die Funktion aktiviert, ist in der Anzeige des Hustengeräts 22 eine Kurvensicht des Beatmungsgeräts 12 sichtbar. Die Kurvensicht auf dem Beatmungsgerät 12 zeigt insbesondere keine Kurven an, sondern verweist auf die aktive Funktion und dass die Inhalte auf dem Hustengerät 22 gezeigt werden.

Wenn die Geräte 1 zum Beispiel entkoppelt werden, sind die Anzeigeeinrichtungen 5 beider Geräte 1 aktiv. In beiden Geräten 1 verschwindet das Kopplungssymbol. In beiden Geräten 1 erscheint eine Benachrichtigung, dass die Verbindung entkoppelt wurde.

Die vorliegende Erfindung bietet eine Kopplung / Interaktion von Beatmungs- und Hustengerät, die bislang unabhängig voneinander genutzt werden. Um die Nutzung zu verbessern, kann ein Gerät 1 in den Standby geschaltet werden, sobald die Nutzung des jeweils anderen Geräts 1 erkannt wird. Zudem wird eine Synchronisierung von Daten bei bspw. mobiler Anwendung der Geräte ermöglicht. Es wird ein besseres Handling und eine erweiterte Funktionalität beider Geräte 1 erreicht, wenn diese gekoppelt sind.

## Patentansprüche

1. Atemtherapiesystem (100) mit wenigstens zwei separaten und unabhängig voneinander betreibbaren Atemtherapiegeräten (1), wobei die Atemtherapiegeräte (1) jeweils wenigstens eine Steuereinrichtung (2) und jeweils wenigstens eine mittels der Steuereinrichtung ansteuerbare Gerätekomponente (3) zur Bereitstellung wenigstens einer Gerätefunktion aufweisen, wobei wenigstens eine Koppeleinrichtung (4) zur Herstellung einer Wirkverbindung zwischen den wenigstens zwei Atemtherapiegeräten (1) vorgesehen ist und wobei die Koppeleinrichtung (4) dazu geeignet und ausgebildet ist, bei hergestellter Wirkverbindung die Steuereinrichtungen (2) der wenigstens zwei Atemtherapiegeräte (1) anzusteuern und wobei mittels der Koppeleinrichtung (3) wenigstens eine Gerätekomponente (3) des wenigstens einen Atemtherapiegerätes (1) durch wenigstens eine Gerätekomponente (3) des wenigstens einen anderen Atemtherapiegerätes (1) wenigstens teilweise funktionell erweiterbar und/oder wenigstens teilweise funktionell ersetzbar ist, wobei das Atemtherapiesystem (1) wenigstens ein als Beatmungsgerät (12) ausgebildetes Atemtherapiegerät (1) und wenigstens ein als Hustengerät (22) ausgebildetes Atemtherapiegerät (1) umfasst, wobei das Beatmungsgerät (12) eine Therapieeinrichtung (7) , umfasst welche als Beatmungseinrichtung (17) ausgebildet ist, wobei die Beatmungseinrichtung (17) mit zumindest einer Gebläseeinrichtung (67) und/oder einem Ventil ausgestattet ist, wobei das Hustengerät (22) zur gezielten Unterstützung einer Sekretabfuhr aus den Atemwegen eines Patienten eingerichtet ist und eine als Husteneinrichtung (27) ausgebildete Therapieeinrichtung (7) umfasst, wobei die Husteneinrichtung (27) zumindest eine Gebläseeinrichtung (67) und ergänzend zumindest ein Ventil umfasst, **dadurch gekennzeichnet, dass** wenigstens eine Anzeigeeinrichtung (5) des wenigstens einen Atemtherapiegerätes (1) durch wenigstens eine Anzeigeeinrichtung (5) des wenigstens einen anderen Atemtherapiegerätes (1) wenigstens teilweise funktionell erweitert und/oder ersetzt wird , sodass eine Datenvisualisierung erweitert bzw. ersetzt wird.

2. Atemtherapiesystem (1) nach dem vorhergehenden Anspruch, wobei die Anzeigeeinrichtungen (5) der wirkverbundenen Atemtherapiegeräte (1) jeweils unter Berücksichtigung einer räumlichen Lage des Atemtherapiegerätes (1) ausrichtbar sind.

3. Atemtherapiesystem (1) nach einem der vorhergehenden Ansprüche, wobei wenigstens eine Bedieneinrichtung (6) des wenigstens einen Atemtherapiegerätes (1) durch wenigstens eine Bedieneinrichtung (6) des wenigstens einen anderen Atemtherapiegerätes (1) wenigstens teilweise funktionell erweiterbar und/oder ersetzbar ist und wobei von der Bedieneinrichtung (6) des wenigstens einen Atemtherapiegerätes (1) aus eine Therapieeinrichtung (7) des wenigstens einen anderen Atemtherapiegerätes (1) ansteuerbar ist und wobei die Therapieeinrichtung (7) insbesondere wenigstens eine Beatmungseinrichtung (17) und/oder wenigstens eine Husteneinrichtung (27) umfasst.

4. Atemtherapiesystem (1) nach einem der vorhergehenden Ansprüche, wobei wenigstens eine Therapieeinrichtung (7) des wenigstens einen Atemtherapiegerätes (1) durch wenigstens eine Therapieeinrichtung (7) des wenigstens einen anderen Atemtherapiegerätes (1) wenigstens teilweise funktionell erweiterbar und/oder ersetzbar ist.

5. Atemtherapiesystem (1) nach dem vorhergehenden Anspruch, Therapieeinrichtung (7) des wenigstens einen Atemtherapiegerätes (1) mit der Therapieeinrichtung (7) des wenigstens einen anderen Atemtherapiegerätes (1) wenigstens unter Berücksichtigung wenigstens eines Geräteparameters und/oder Beatmungsparameters synchronisierbar ist.

6. Atemtherapiesystem (1) nach einem der zwei vorhergehenden Ansprüche, wobei die Therapieeinrichtung (7) des wenigstens einen Atemtherapiegerätes (1) dazu geeignet und ausgebildet ist, mittels der Koppeleinrichtung (4) wenigstens eine in dem wenigstens einen anderen Atemtherapiegerätes (1) angeordnete Verneblereinrichtung (37) anzusteuern.

7. Atemtherapiesystem (1) nach einem der drei vorhergehenden Ansprüche, wobei die Therapieeinrichtung (7) des wenigstens einen Atemtherapiegerätes (1) dazu geeignet und ausgebildet ist, mittels der Koppeleinrichtung (4) ein Ausatemsystem (47) des wenigstens einen anderen Atemtherapiegerätes (1) anzusteuern und insbesondere pneumatisch anzusteuern.

8. Atemtherapiesystem (1) nach einem der vier vorhergehenden Ansprüche, wobei die Therapieeinrichtung (7) des wenigstens einen Atemtherapiegerätes (1) mit wenigstens einem Exspirationsteil des anderen Atemtherapiegerätes (1) zur Herstellung einer Strömungsverbindung verbindbar ist, um bei hergestellter Wirkverbindung als eine Unterdruckeinheit für die Ausatmung in der Exspiration zu dienen.

9. Atemtherapiesystem (1) nach einem der vorhergehenden Ansprüche, wobei die Koppeleinrichtung (4) dazu geeignet und ausgebildet ist, die Gerätekomponente (3) des wenigstens einen Atemtherapiegerätes (1) und/oder die Gerätekomponente (3) des wenigstens einen anderen Atemtherapiegerätes (1) wenigstens teilweise zu deaktivieren und/oder zu aktivieren, wenn die Atemtherapiegeräte (1) wirkverbunden sind.

10. Atemtherapiesystem (1) nach einem der vorhergehenden Ansprüche, wobei wenigstens eine Aufnahmeeinrichtung (8) zur physischen Aufnahme der wenigstens zwei Atemtherapiegeräte (1) umfasst ist und wobei die Aufnahmeeinrichtung (8) für die Atemtherapiegeräte (1) jeweils wenigstens eine Aufnahmeeinheit (18) aufweist und/oder wobei die Aufnahmeeinrichtung (8) für die Atemtherapiegeräte (1) wenigstens eine gemeinsame Aufnahmeeinheit (28) aufweist.

11. Atemtherapiesystem (1) nach zumindest einem der vorhergehenden Ansprüche, wobei die Atemtherapiegeräte (1) in der Aufnahmeeinrichtung (8) so angeordnet sind, dass die Anzeigeeinrichtung (5a) des einen Atemtherapiegerätes (1a) in einem Winkel (a) zu einer Horizontalen (h) geneigt angeordnet ist und dieser Winkel im Bereich 4° bis 45° liegt und wobei die Anzeigeeinrichtung (5b) des anderen Atemtherapiegerätes (1b) in einem Winkel (b) zu einer Vertikalen (v) geneigt angeordnet ist und dieser Winkel im Bereich 4° bis 45° liegt.

12. Atemtherapiesystem (1) nach zumindest einem der vorhergehenden Ansprüche, wobei die Atemtherapiegeräte (1) in der Aufnahmeeinrichtung (8) so angeordnet sind, dass die Anzeigeeinrichtungen 5a und 5b einen Winkel (c) zueinander aufweisen, der größer als 90° ist und bevorzugt bei 92° bis 172° liegt.

13. Atemtherapiesystem (1) nach zumindest einem der vorhergehenden Ansprüche, wobei die Atemtherapiegeräte (1) in der Aufnahmeeinrichtung (8) so angeordnet sind, dass die Unterkante einer Anzeigeeinrichtung im Wesentlichen auf derselben Höhe ist wie die Oberkante der anderen Anzeigeeinrichtung.

## Claims

1. A breathing therapy system (100) with at least two separate and independently operable breathing therapy devices (1), wherein the breathing therapy devices (1) each have at least one control apparatus (2) and each have at least one device component (3) that can be controlled by means of the control apparatus for providing at least one device function, wherein at least one coupling apparatus (4) is provided for establishing an operative connection between the at least two breathing therapy devices (1), and wherein the coupling apparatus (4) is suitable and designed to control the control apparatuses (2) of the at least two breathing therapy devices (1) when the operative connection is established, and wherein, by means of the coupling apparatus (3), at least one device component (3) of the at least one breathing therapy device (1) can be at least partially functionally expanded and/or at least partially functionally replaced by at least one device component (3) of the at least one other breathing therapy device (1), wherein the breathing therapy system (1) comprises at least one breathing therapy device (1) designed as a ventilator (12) and at least one breathing therapy device (1) designed as a cough device (22), wherein the ventilator (12) comprises a therapy apparatus (7) which is designed as a ventilation apparatus (17), wherein the ventilation apparatus (17) is equipped with at least one blower apparatus (67) and/or a valve, wherein the cough device (22) is configured for targeted support of discharging secretions from the airways of a patient and comprises a therapy apparatus (7) designed as a cough apparatus (27), wherein the cough apparatus (27) comprises at least one blower apparatus (67) and additionally at least one valve, **characterized in that** at least one display apparatus (5) of the at least one breathing therapy device (1) is at least partially functionally expanded and/or replaced by at least one display apparatus (5) of the at least one other breathing therapy device (1) such that a data visualization is expanded or replaced, respectively.

2. The breathing therapy system (1) according to the preceding claim, wherein the display apparatuses (5) of the operatively connected breathing therapy devices (1) can each be oriented considering a spatial position of the breathing therapy device (1).

3. The breathing therapy system (1) according to one of the preceding claims, wherein at least one operating apparatus (6) of the at least one breathing therapy device (1) can be at least partially functionally expanded and/or replaced by at least one operating apparatus (6) of the at least one other breathing therapy device (1), and wherein a therapy apparatus (7) of the at least one other breathing therapy device (1) can be controlled from the operating apparatus (6) of the at least one breathing therapy device (1), and wherein the therapy apparatus (7) comprises in particular at least one ventilation apparatus (17) and/or at least one cough apparatus (27).

4. The breathing therapy system (1) according to one of the preceding claims, wherein at least one therapy apparatus (7) of the at least one breathing therapy device (1) can be at least partially functionally expanded and/or replaced by at least one therapy apparatus (7) of the at least one other breathing therapy device (1).

5. The breathing therapy system (1) according to the preceding claim, wherein the therapy apparatus (7) of the at least one breathing therapy device (1) can be synchronized with the therapy apparatus (7) of the at least one other breathing therapy device (1) by means of the coupling apparatus (4) at least considering at least one device parameter and/or ventilation parameter.

6. The breathing therapy system (1) according to one of the two preceding claims, wherein the therapy apparatus (7) of the at least one breathing therapy device (1) is suitable and designed to control, by means of the coupling apparatus (4), at least one nebulizer apparatus (37) arranged in the at least one other breathing therapy device (1).

7. The breathing therapy system (1) according to one of the three preceding claims, wherein the therapy apparatus (7) of the at least one breathing therapy device (1) is suitable and designed to control and in particular pneumatically control, by means of the coupling apparatus (4), an exhalation system (47) of the at least one other breathing therapy device (1).

8. The breathing therapy system (1) according to one of the four preceding claims, wherein the therapy apparatus (7) of the at least one breathing therapy device (1) can be connected to at least one expiration part of the other breathing therapy device (1) to establish a flow connection in order to serve in the expiration as a negative pressure unit for the exhalation when the operative connection is established.

9. The breathing therapy system (1) according to one of the preceding claims, wherein the coupling apparatus (4) is suitable and designed to at least partially deactivate and/or activate the device component (3) of the at least one breathing therapy device (1) and/or the device component (3) of the at least one other breathing therapy device (1) when the breathing therapy devices (1) are operatively connected.

10. The breathing therapy system (1) according to one of the preceding claims, wherein at least one accommodation apparatus (8) is comprised to physically accommodate the at least two breathing therapy devices (1) and wherein the accommodation apparatus (8) has at least one accommodation unit (18) for each of the breathing therapy devices (1) and/or wherein the accommodation apparatus (8) has at least one common accommodation unit (28) for the breathing therapy devices (1).

11. The breathing therapy system (1) according to at least one of the preceding claims, wherein the breathing therapy devices (1) are arranged in the accommodation apparatus (8) such that the display apparatus (5a) of the one breathing therapy device (1a) is arranged tilted at an angle (a) to a horizontal (h) and this angle is in the range 4° to 45°, and wherein the display apparatus (5b) of the other breathing therapy device (1b) is arranged tilted at an angle (b) to a vertical (v) and this angle is in the range of 4° to 45°.

12. The breathing therapy system (1) according to at least one of the preceding claims, wherein the breathing therapy devices (1) are arranged in the accommodation apparatus (8) such that the display apparatuses 5a and 5b have an angle (c) to each other that is greater than 90° and is preferably at of 92° to 172°.

13. The breathing therapy system (1) according to at least one of the preceding claims, wherein the breathing therapy devices (1) are arranged in the accommodation apparatus (8) such that the bottom edge of one display apparatus is basically at the same height as the top edge of the other display apparatus.

## Revendications

1. Système de thérapie respiratoire (100) comprenant au moins deux appareils de thérapie respiratoire (1) séparés et aptes à fonctionner indépendamment l'un de l'autre, dans lequel les appareils de thérapie respiratoire (1) présentent respectivement au moins un dispositif de commande (2) et respectivement au moins un composant d'appareil (3) apte à être commandé au moyen du dispositif de commande (2) pour fournir au moins une fonction d'appareil, dans lequel il est prévu au moins un dispositif d'accouplement (4) permettant d'établir une liaison fonctionnelle entre les au moins deux appareils de thérapie respiratoire (1) et dans lequel le dispositif d'accouplement (4) est adapté et conçu pour commander les dispositifs de commande (2) des au moins deux appareils de thérapie respiratoire (1) lorsque la liaison fonctionnelle est établie et dans lequel le dispositif d'accouplement (3) permet de développer et/ou de remplacer au moins partiellement au moins un composant d'appareil (3) de l'au moins un appareil de thérapie respiratoire (1) fonctionnellement par au moins un composant d'appareil (3) de l'au moins un autre appareil de thérapie respiratoire (1), dans lequel le système de thérapie respiratoire (1) comporte au moins un appareil de thérapie respiratoire (1) conçu comme un appareil d'assistance respiratoire (12) et au moins un appareil de thérapie respiratoire (1) conçu comme un appareil de toux (22), dans lequel l'appareil d'assistance respiratoire (12) comporte un dispositif thérapeutique (7) conçu comme un dispositif d'assistance respiratoire (17), dans lequel le dispositif d'assistance respiratoire (17) est équipé d'au moins un dispositif de ventilation (67) et/ou d'une soupape, dans lequel l'appareil de toux (22) est configuré pour soutenir de façon ciblée une évacuation de sécrétions hors des voies respiratoires d'un patient et comporte un dispositif thérapeutique (7) conçu comme un dispositif de toux (27), dans lequel le dispositif de toux (27) comporte au moins un dispositif de ventilation (67) et au moins une soupape complémentaire, **caractérisé en ce qu'**au moins un dispositif d'affichage (5) de l'au moins un appareil de thérapie respiratoire (1) est au moins partiellement développé et/ou remplacé fonctionnellement par au moins un dispositif d'affichage (5) de l'au moins un autre appareil de thérapie respiratoire (1), de manière à développer ou remplacer une visualisation de données.

2. Système de thérapie respiratoire (1) selon la revendication précédente, dans lequel les dispositifs d'affichage (5) des appareils de thérapie respiratoire (1) fonctionnellement reliés peuvent être orientés respectivement en fonction d'un emplacement spatial de l'appareil de thérapie respiratoire (1).

3. Système de thérapie respiratoire (1) selon l'une des revendications précédentes, dans lequel au moins un dispositif d'actionnement (6) de l'au moins un appareil de thérapie respiratoire (1) peut être au moins partiellement développé et/ou remplacé fonctionnellement par au moins un dispositif d'actionnement (6) de l'au moins un autre appareil de thérapie respiratoire (1) et dans lequel le dispositif d'actionnement (6) de l'au moins un appareil de thérapie respiratoire (1) permet de commander un dispositif thérapeutique (7) de l'au moins un autre appareil de thérapie respiratoire (1) et dans lequel le dispositif thérapeutique (7) comporte en particulier au moins un dispositif d'assistance respiratoire (17) et/ou au moins un dispositif de toux (27).

4. Système de thérapie respiratoire (1) selon l'une des revendications précédentes, dans lequel au moins un dispositif thérapeutique (7) de l'au moins un appareil de thérapie respiratoire (1) peut être au moins partiellement développé et/ou remplacé fonctionnellement par au moins un dispositif thérapeutique (7) de l'au moins un autre appareil de thérapie respiratoire (1).

5. Système de thérapie respiratoire (1) selon la revendication précédente, dans lequel le dispositif d'accouplement (4) permet de synchroniser le dispositif thérapeutique (7) de l'au moins un appareil de thérapie respiratoire (1) avec le dispositif thérapeutique (7) de l'au moins un autre appareil de thérapie respiratoire (1) au moins en fonction d'au moins un paramètre d'appareil et/ou paramètre d'assistance respiratoire.

6. Système de thérapie respiratoire (1) selon l'une des deux revendications précédentes, dans lequel le dispositif thérapeutique (7) de l'au moins un appareil de thérapie respiratoire (1) est adapté et conçu pour commander au moins un dispositif de nébulisation (37) disposé dans l'au moins un autre appareil de thérapie respiratoire (1) au moyen du dispositif d'accouplement (4).

7. Système de thérapie respiratoire (1) selon l'une des trois revendications précédentes, dans lequel le dispositif thérapeutique (7) de l'au moins un appareil de thérapie respiratoire (1) est adapté et conçu pour commander et en particulier pour commander de façon pneumatique un système expirateur (47) de l'au moins un autre appareil de thérapie respiratoire (1) au moyen de dispositif d'accouplement (4).

8. Système de thérapie respiratoire (1) selon l'une des quatre revendications précédentes, dans lequel le dispositif thérapeutique (7) de l'au moins un appareil de thérapie respiratoire (1) peut être relié à au moins une partie d'expiration de l'autre appareil de thérapie respiratoire (1) pour établir une liaison de flux, afin de servir d'unité de dépression pour le souffle sortant pendant l'expiration lorsque la liaison fonctionnelle est établie.

9. Système de thérapie respiratoire (1) selon l'une des revendications précédentes, dans lequel le dispositif d'accouplement (4) est adapté et conçu pour désactiver et/ou pour activer au moins partiellement le composant d'appareil (3) de l'au moins un appareil de thérapie respiratoire (1) et/ou le composant d'appareil (3) de l'au moins un autre appareil de thérapie respiratoire (1), lorsque les appareils de thérapie respiratoire (1) sont reliés fonctionnellement.

10. Système de thérapie respiratoire (1) selon l'une des revendications précédentes, comportant au moins un dispositif de réception (8) destiné à recevoir physiquement les au moins deux appareils de thérapie respiratoire (1) et dans lequel le dispositif de réception (8) présente au moins une unité de réception (18) respectivement pour les appareils de thérapie respiratoire (1) et/ou dans lequel le dispositif de réception (8) présente au moins une unité de réception commune (28) pour les appareils de thérapie respiratoire (1).

11. Système de thérapie respiratoire (1) selon l'une au moins des revendications précédentes, dans lequel les appareils de thérapie respiratoire (1) sont disposés de telle façon dans le dispositif de réception (8), que le dispositif d'affichage (5a) de l'un des appareils de thérapie respiratoire (1a) est disposé de façon inclinée selon un angle (a) par rapport à une horizontale (h) et cet angle est compris entre 4° et 45° et dans lequel le dispositif d'affichage (5b) de l'autre appareil de thérapie respiratoire (1b) est disposé de façon inclinée selon un angle (b) par rapport à une verticale (v) et cet angle est compris entre 4° et 45°.

12. Système de thérapie respiratoire (1) selon l'une au moins des revendications précédentes, dans lequel les appareils de thérapie respiratoire (1) sont disposés de telle façon dans le dispositif de réception (8), que les dispositifs d'affichage 5a et 5b présentent un angle (c) entre eux, lequel est supérieur à 90° et de préférence compris entre 92° et 172°.

13. Système de thérapie respiratoire (1) selon l'une au moins des revendications précédentes, dans lequel les appareils de thérapie respiratoire (1) sont disposés de telle façon dans le dispositif de réception (8), que le bord inférieur d'un dispositif d'affichage se trouve quasiment à la même hauteur que le bord supérieur de l'autre dispositif d'affichage.
